# EUROPEAN PATENT APPLICATION

(11) **EP 2 002 862 A1**
(43) Date of publication of application: **17.12.2008**
(21) Application number: 07252389.7
(22) Date of filing: 13.06.2007
(51) Int. Cl.: A61N 1/36, A61N 1/04, A61H 39/00, A61M 21/02, A61M 21/00

(54) **Alpha-wave inducer**

(71) Applicant: Jeong, Chong-Phil, Youngdeungpo-Ku Seoul 150-936 (KR)
(72) Inventor: Jeong, Chong-Phil, Youngdeungpo-Ku Seoul 150-936 (KR)
(74) Representative: Neobard, William John

(57) **Abstract**

An alpha-wave inducer characterized in that frequency is selected from the range 1 Hz to 50 Hz, preferably, 7 Hz to 14 Hz, and a voltage selected from the range of 0 mV to 100 mV and is applied to auricle of a patient's ears to induce alpha-waves, and the cycle and intensity of the stimulation are varied depending upon the body temperature and blood sugar level.

## Description

The present invention relates to an alpha-wave inducer. Embodiments relate to an alpha-wave inducer for applying electric stimulation to an auricle of vagus nerves of an ear with the aim of inducing alpha-waves in a brain wave.

An electric stimulator for vagus nerves can, with approval from the Food and Drugs Administration, be used for treating epilepsy and hypochondria to the cervix of the nerves. It has also been reported that the electric stimulator is effective for treatment of obesity in epileptics. The vagus nerves serve as transferring paths, which transfer arousal information from lower organs to the cerebrum. Since the vagus nerves are frequency dependent, it has been possible to induce synchronous waves and asynchronous brain waves (see "Vagus Nerve Stimulation" by Steven C. Schachter and Clifford B. Saper: Epilepsia 39: 682, 1998).

Electric stimulation therapy of the cervix of vagus nerves is performed by removing skin to expose the cervix of the vagus nerves, winding a coil around the cervix, and grafting a microchip therein. However, operation costs are very high, and the region must be operated on every few years in order to replace the battery. In addition, a permanent scar is left due to the incision. Furthermore, the patient is subjected to the mental stress and normal risks of an operation.

The oriental practice of acupuncture to the ear, although effective for obesity, pain and internal organ disease due, substantially, to stimulation of the vagus nerves, places special stress on energy and meridians which may counter the effect of stimulation via nerves. Equally, since oriental medicine does not acknowledge that the simulation conducting effect of the vagus nerves is different depending upon the frequency by electrical synapse of the vagus nerves, effective treatment is not performed. In addition, a patient must go to hospital every day so that acupuncture may be performed. Since a chronic disease results from the reduction or blunting of regulation of the vagus nerves, a patient's symptoms relapse when acupuncture is not performed. Therefore, the patient's life is encumbered, because of frequent visits to hospital throughout their life.

Embodiments of the invention address the above problems and provide an alpha-wave inducer capable of implementing the following functions:
1. The alpha-wave is induced by selecting auricles of both ears in which only the auricle among vagus nerves known as having a part in the synchronization of brain waves is positioned near to the skin. By effectively implementing the stimulation of a specific frequency and intensity, treating various chronic diseases that result from reduction or blunting of regulation of vagus nerves, thereby increasing the effect on various diseases such as obesity and glycosuria. Furthermore, the patient's power of concentration is increased by relieving mental tension and fatigue, reducing stress to maintain the optimum brain state and to enhance memory and thinking power;
2. Cymba conchae (100%), which is a prominent region of vagus nerves and a front surface (50%) and rear surface (50%) of auricle concha may be simultaneously stimulated, and the effect of the treatment may be retained without causing the patient inconvenience in daily life such as walking or exercise. According to embodiments of the invention, a unit for detecting the detachment of a stimulating pad from a stimulating point of the patient is provided. In an embodiment, the unit is designed so that the detachment or noticeability of others is reduced;
3. In order to minimize wearing sensitivity, in an embodiment of the invention, the alpha-wave inducer is of low electric power consumption and developed as a circuit of digital mode. With this development, the intensity and cycle of the stimulating signal may conveniently be adjusted by the patient;
4. Being different from a conventional alpha-wave inducer, embodiments of the alpha-wave inducer provide a transmitter and receiver that are separately divided, thereby minimizing the dimensions of the inducer. The inducer may be continuously attached to the body to continuously induce alpha-waves; and
5. Considering conditions of the patient at use, i.e., humidity and contacting state of a surface to be attached, in embodiments stimulation is controlled, and may be controlled according to a monitored body temperature and/or blood sugar level.

According to an aspect of the present invention, there is provided an alpha-wave inducer comprising: a main control unit for generating a low frequency signal of frequency and cycle selected from a range of 1 Hz to 50 Hz and a voltage selected from a range of 0 mV to 100 mV to induce an alpha-wave; and an electric stimulating unit for applying the low frequency signal generated in the main control unit to an auricle of vagus nerves of an ear.

The main control unit may generate a low frequency signal of frequency and cycle selected from a range of 7 Hz to 14 Hz and a voltage selected from a range of 1 mV to 20 mV

According to another aspect of the present invention, there is provided an alpha-wave inducer comprising: a battery for supplying an electric power to the entire of a system; a voltage converter for converting the electric power from the battery into a voltage of a predetermined value set at 0 mV to 100 mV; a low frequency generator for generating a low frequency of a predetermined value set at 1 Hz to 50 Hz based on the voltage from the voltage converter; a button input unit including an electric power/function button and a set button; a display for displaying a value set by an operation of the button input unit; a microprocessor for controlling a frequency of the low frequency generator and a voltage of the voltage converter based on the value set by the operation of the button input unit; and an electric stimulating unit including a plurality of contact terminals for applying the low frequency generated by the low frequency generator to an auricle of an ear.

Embodiments of the Invention will now be described by way of example only with reference to the figures wherein:
Fig. 1 is a perspective view illustrating the construction of an alpha-wave inducer embodying the present invention.
Fig. 2 is a view depicting a state of use of an electric stimulating unit.
Fig. 3 is a schematic view depicting a main control unit of an alpha-wave inducer according to one preferred embodiment.
Figs. 4a to 4c are flowcharts depicting a stimulating method according to body temperature and/or blood sugar.
Figs. 5a to 5f are graphs quantitatively showing distribution of alpha waves, beta waves, gamma waves, delta waves, and theta waves before using an alpha-wave inducer embodying the present invention.
Figs. 6a to 6f are graphs quantitatively showing distribution of alpha waves, beta waves, gamma waves, delta waves, and theta waves after use of an alpha-wave inducer embodying the present invention.
Figs. 7a and 7b are views illustrating the state which alpha power is increased in a head before and after use of an alpha-wave inducer embodying to the present invention.
Figs. 8a and 8b and Figs. 9a and 9b are views illustrating the state which synchronization is increased in a forebrain before and after use of an alpha-wave inducer embodying to the present invention.
Fig. 10a is a view depicting an alpha-wave inducer according to another embodiment.
Fig. 10b is a view depicting the state where a user puts on an alpha-wave inducer implemented to have an ear ring shape according to another embodiment.
Fig. 10c is a view depicting the state where the user puts on an alpha-wave inducer implemented to have a necklace shape according to another embodiment.
Fig. 11 is a view illustrating the internal construction of an alpha-wave inducer according to another embodiment.

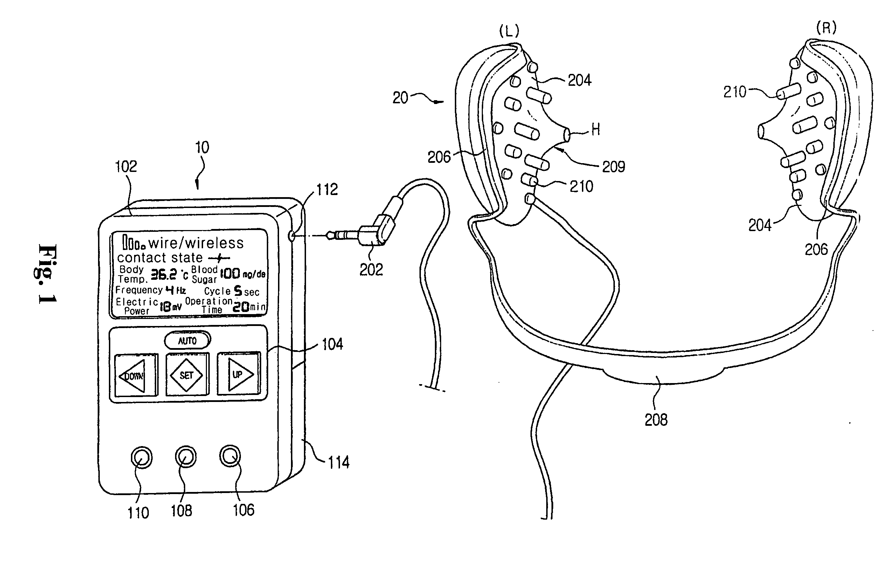

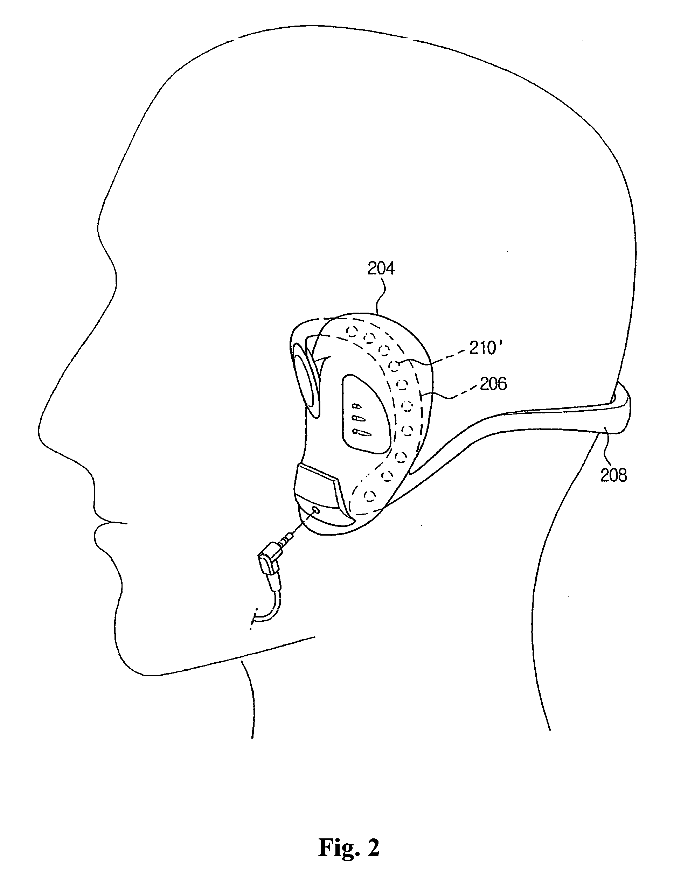

Referring to Figs. 1 and 2, the alpha-wave inducer includes a main control unit 10 and an electric stimulating unit 20.

The main control unit 10 is provided at a front thereof with a display 102, setting keys 104, a power input button 106, a wire/wireless mode indicating lamp 108, and a touch state indicating lamp 110. The display 102 displays a contact state, body temperature, and blood sugar level, as well as frequency, voltage, period, operating time and the like, which are set by the user. Also, the display 102 displays a residual amount of electric power and wired/wireless mode. The setting keys consist of a mode select key "SET", up/down control keys "UP" and "DOWN", and an auto-mode key "AUTO". The main control unit 10 is provided at one side thereof with a connecting terminal 112 connected to a jack 202 of the electric stimulating unit 20. A battery case 114 is mounted to a rear lower end of the main control unit 10 to accommodate a battery.

The electric stimulating unit 20 is provided as a headphone shape in the embodiment. The electric stimulating unit 20 includes left and right stimulating member L and R each having a front stimulating portion 204 and a rear stimulating portion 206 to effectively apply electric stimulation to user's ears. The left stimulating member L is connected to the right stimulating member R via a connecting band 208. The front stimulating portion 204 is formed in an ear shape so as to selectively apply the electric stimulation to the front side of the ear. The rear stimulating portion 206 is formed in a shape to enclose a rear side of the ear so as to effectively apply the electric stimulation to the rear side of the ear. In a modification, the electric stimulating unit 20 may be formed in an acousticon shape for stimulating cymba conchae and a front surface of auricle concha or an earphone shape for stimulating the front surface of the auricle concha or a spectacular's leg inducer shape for stimulating a rear surface of the auricle concha or a decoration shape which is not distinguishable from general earrings or a connector for inducer at both front and rear surfaces of the auricle concha the like, so as to stimulate the front surface (50%) of the auricle concha and a rear surface (50%) thereof, as well as the cymba conchae (100%) which is a dominant region of auricle of vagus nerves. The skilled person will be aware of other shapes. These types of inducers may effectively induce the alpha-waves to undergo medical treatment of diseases, without being unhandy.

The close contact to the ear is achieved by the front stimulating portion 204 and the rear stimulating portion 206. The front stimulating portion 204 is provided with a body temperature/blood sugar detecting portion 209 for detecting the body temperature and the blood sugar level. Specifically, the body portion 209 has a shape having a gently slant ridgeline, such as a crater, so as to be easily inserted in an external auditory meatus. The body portion 209 has a drilled top surface H to receive an infrared signal.

The front and rear stimulating portions 204 of the left and right stimulating member L and R, comprising the electric stimulating unit 20, are provided with a plurality of electric rods 210 and 210', considering a general shape of the ear, i.e., a shape and depth of the concha. The number of electric rods 210 and 210' may be adjusted, if necessary. An end of the respective electric rods 210 and 210' is rounded. The length of the respective electric rods is may be different to each other, in consideration of the depth of the concha. The electric rod 210 of the front stimulating member 210 may be constructed in such a manner that its length can be adjusted. The electric rod 210' of the rear stimulating member 206 may be is made of a semicircle metal piece.

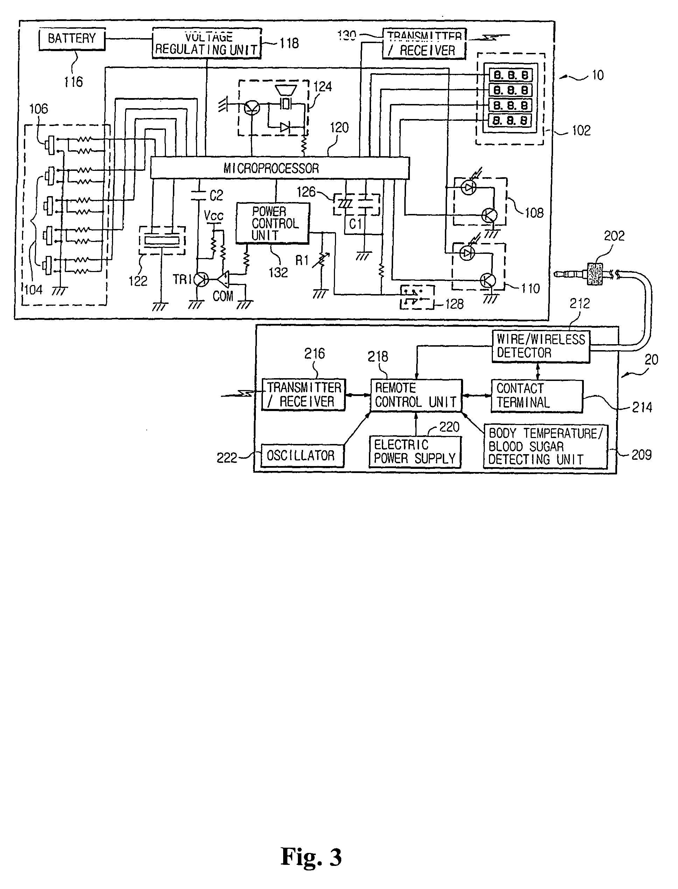

Fig. 3 is a schematic view depicting a main control unit of an alpha-wave inducer according to an embodiment of the present invention. As shown in Fig. 3, the main control unit includes a battery 116 for supplying an electric power to the entire of the system, a voltage regulating unit 118 for transforming a voltage outputted from the battery 116 to a constant voltage corresponding to one required for the system, and a microprocessor 120 receiving the electric power from the voltage regulating unit 118 for initializing and controlling the system. The microprocessor 120 is provided with an A/D converter (not shown) and a D/A converter (not shown) for processing input and output signals. The microprocessor 120 is connected to the key input supporting key operation of a user. The key input consists of the mode selecting key "SET", the up/down control keys "UP" and "DOWN", the auto-mode key "AUTO", and the power input key 106. The microprocessor 120 includes an oscillator 122 for carrying out a frequency oscillation selected by a user, an alarm 124 for alerting whether or not the electric rods 210 and 210' are contacted to the user's ears or alerting if the user is perspiring or notifying completion of the operation time; a non-contact detector 126 connected to the electric stimulating unit 20 for detecting the contact state of the electric rods 210 and 210'; and a connector 128 connected to the non-contact detector 126 for transferring a corresponding frequency and voltage to the non-contact detector and transferring the contact state of the electric rods 210 and 210' to the non-contact detector 126, according to a control of the microprocessor 120. A D/A converter (not shown) is interposed between the connector 128 and the microprocessor 120. In particular, the D/A converter is built in the microprocessor 120. Display 102 and a LCD are provided to display a frequency, a voltage, a period, an operation time, a contact state, body temperature, blood sugar level and the like. Specifically, the frequency and voltage are set in a range of 1 Hz to 50 Hz and 0 mV to 100 mV, respectively, and a corresponding value is displayed. The period is a time interval which the frequency and voltage are applied to the ears, and is displayed in the unit of seconds. The operation time is displayed in the unit of minutes. The LED is provided to visually notify the use state. The touch state indicating lamp 110, which cooperates with a function of the alarm 124, alerts the completion of the operation time and the contact state of the electric rods 210 and 210' The wire/wireless mode indicating lamp 108 displays the state of the wire/wireless mode and is provided as the LED. The microprocessor 120 includes a transmitter/receiver 130 for transmitting control input data of the user and receiving body temperature and blood sugar data. The transmitter/receiver wirelessly controls the main control unit 10 and the electric stimulating unit 20.

The electric stimulating unit 20 is connected to the jack 202, inserted in the connector 128, for applying the electric stimulation to the ears and includes a wire/wireless detector 212 for detecting whether the jack 202 is connected to the connector 128 in order to operate the electric stimulating unit in a wireless mode. A contact terminal 214 is connected to the wire/wireless detector 212 to be contacted with the electric rods 210 and 210' contacted to the ears. The contact terminal 214 may be connected to the electric rods 210 and 210', or the contact terminal 214 itself may perform the function of the electric rods 210 and 210'. In addition, when operating in the wireless mode, the electric stimulating unit 20 includes a transmitter/receiver 216 for receiving the signal transmitted to the microprocessor 120 of the main control unit and transmitting the data of the checked body temperature and blood sugar level; a remote control unit 218 for controlling the data transmitted from the transmitter/receiver 216; an electric power supply 220 for supplying the electric power to the remote control unit 218 and the contact terminal 214; and an oscillator 222 for oscillating the corresponding frequency to output the corresponding frequency and voltage to the contact terminal 214. The transmitter/receiver 216, the oscillator 222 and the electric power supply 220 operate in wireless mode only.

When the wire mode is selected (by a key operation of the mode select key SET of the setting keys 104 or the wire mode is selected by insertion of the jack 202 in the connector 128 in the state where the auto-mode key AUTO is key-operated) if a frequency and a voltage wanted by the user are selected by a key operation of the up/down control keys UP and DOWN of the setting keys 104, the microprocessor 120 of the main control unit 10 controls the oscillator 122 to generate the selected frequency and also controls the electric power control unit 132 to generate the selected voltage. Here, in order to induce alpha-waves in a range of 7 Hz to 14 Hz, preferably, a frequency in a range of 7 Hz to 14 Hz is selected by the user and preferably, a voltage in a range of 1 mV to 20 mV is selected.

That is, when the user selects the frequency in a range of 7 Hz to 14 Hz and the voltage in a range of 1 mV to 20 mV so as to induce alpha-waves, the main control unit 10 generates a frequency selected from a range of 7 Hz to 14 Hz in the oscillator 122 in the wire mode state and the electric power control unit 132 generates a voltage selected from a range of 1 mV to 20 mV through the connector 128. As such, the frequency and the voltage are applied to the contact terminals 214 through the jack 202 connected to the connector 128 and electric stimulation is applied to an auricle of the vagus nerves of an ear through the electric rods 210 and 210' contacted to the contact terminals 214 so that alpha-waves in a range of 7 Hz to 14 Hz can be induced.

In addition, when the wireless mode is selected (by a key operation of the mode select key SET of the setting keys 104 or the wireless mode is selected by detecting that the jack 202 is not inserted in the connector 128 using the wire/wireless detector 212 in the state where the auto-mode key AUTO is key-operated) if a frequency and a voltage wanted by the user are selected by a key operation of the up/down control keys UP and DOWN of the setting keys 104, the microprocessor 120 wirelessly transmits control input data of the frequency and the voltage selected by the user to the electric stimulating unit 20 via the transmitter/receiver 130 and the remote control unit 218 of the electric stimulating unit 20 receives the control input data from the transmitter/receiver 216 so that a selected frequency can be generated in the oscillator 222 and a selected voltage can be generated in the electric power supply 220 according to the frequency and the voltage selected by the user.

When the user selects the frequency in a range of 7 Hz to 14 Hz and the voltage in a range of 1 mV to 20 mV so as to induce alpha-waves, the electric stimulating unit 20 generates a frequency selected from a range of 7 Hz to 14 Hz in the oscillator 122 in the wireless mode state and the electric power supply 220 generates a voltage selected from a range of 1 mV to 20 mV through the contact terminals 214. As such, electric stimulation is applied to an auricle of the vagus nerves of an ear through the electric rods 210 and 210' contacted to the contact terminals 214 so that alpha-waves in a range of 7 Hz to 14 Hz can be induced.

The non-contact detector 126 detects the contact state of the electric rods 210 and 210'. In the case of the contact state, embodiments of the present invention perform the control in view of the perspiring state of the user, so as to protect the user and the alpha-wave inducer.

Specifically, in order to apply the electric stimulation to the auricle perspiring state of the user, the electrode has to be closely attached to the auricle region. According to the user's skin conditions, in other words, in the case that the skin becomes moist with perspiration or after taking a shower, an electrical short circuit may occur between the electrodes. The electrical short circuit causes a short-circuit current to flow due to a microvoltage applied between the electrodes. Accordingly, an electric power device supplying the electric power to auricle region may be damaged by the short-circuit current. In addition, the electric stimulation to be applied to the auricle region does not apply to the auricle region of the user, so the treatment is not performed.

In order to prevent the above short circuit, the non-contact detector firstly detects the contact state depending upon a quantity of electric charge in a capacitor C1. If the electrodes are contacted, in the case where the microprocessor 120 determines that the electric charge is not charged in the capacitor C1 (a low signal generates), the electric power control unit 132 is activated to supply a direct current through the electrodes of the auricle region. At that time, a direct current of 200 µA is suitable for the current to be supplied, causing a degree of harm to a body, but the current value may be varied in the range which is not harmful to the body, as understood by the skilled reader.

A closed circuit is formed by the electrode of the auricle, the electric rods 210 and 210' contacted to the auricle, and a variable resistor R1 connected to the electric rods. The supplied current flows through the closed circuit. A voltage drop occurs in the closed circuit corresponding to the resultant resistance of the skin resistance of the auricle and the variable resistor R1.

The skin resistance between the electric rods 210 and 210' of the auricle region is typically 25 to 40 kΩ. For example, if the skin resistance 37 kΩ, a voltage drop of typically 0.54 V occurs In that case the auricle is in a state capable of performing the treatment, and the value of voltage drop is applied to a voltage comparator COM. If the voltage comparator COM detects more than 0.5 volt, it generates a high signal. In a normal state, a voltage of more than a set value is applied to the voltage comparator COM, so that an output of the voltage comparator becomes a high. The electric charge in capacitor C2 is discharged through a connected transistor TR1 in response to the high signal of the voltage comparator.

At that time, if the capacitor C2 is not charged, the microprocessor 120 regards the auricle as in a normal state and interrupts the supply of the direct current through the electric power control unit 132 (although not shown, a switch or switching circuit selectively supplying the direct voltage and the electric power is built in the electric power control unit 132, and is operated by the control signal of the microprocessor 120. The operation thereof is widely known in the prior art, and its description is omitted herein), and supplies a normal waveform through the electrode, thereby performing the treatment. If the user is wet and the auricle is in the electrically connected state, the current supplied to the auricle is only voltage dropped by the variable resistor R1.

Although the present invention utilizes a variable resistor of about 3 to 10 kΩ, it may be changed as understood by the skilled reader. At that time, the voltage supplied to the voltage comparator COM is less than 0.5 volts, and the voltage comparator COM changes to the low state. The transistor TR is not connected, and the capacitor C2 is charged. The microprocessor 120 regards the auricle as in a wet state, and outputs the control signal for turning on/off the contact alarm lamp 110.

The electric stimulating unit 20 is provided with the body temperature/blood sugar detecting portion 209. The body temperature/blood sugar detecting portion 209 includes a sensor for detecting infrared radiation naturally emitted from the human body. Specifically, the sensor is an infrared processing module, in which it can stably measure the skin temperature of a patient by determining the infrared ray emitted from a tympanum, with it being inserted in the external auditory meatus. Since the tympanum uses the same blood vessel as that of a hypothalamus, and is a center of body temperature adjustment, it is a proper position to measure the body temperature. A tympanum thermometer utilizes an ear of a user. The thermometer is inserted in the external auditory meatus, so that the detecting unit is sufficiently enclosed. Radiation reflected from the tympanum transforms the external auditory meatus to a blackbody cavity, having an emissivity of 1. With the above description, the sensor exactly radiates the tympanum and its blood vessel to determine a ratio of the infrared radiation emitted by the patient's tympanum. It is applied to the Planck's law which represents a relationship between the radiation intensity, the spectral distribution and a blackbody's temperature. If the temperature rises, the radiation energy increases, and the radiation energy varies depending upon a wavelength. A peak value of radiation is shifted toward a shorter wavelength according to the temperature rise. Radiation happens over a wide band of wavelengths. The entire energy radiated from the blackbody and measured by the non-contact infrared thermometer is the result of the entire energy radiated over all wavelengths. This is proportional to the integral of the Planck's law over every wavelength. It is fully described in the Stefan-Boltzmann's law of the physics.

In addition, the spectral emissivity of the infrared radiation emitted from the tympanum includes spectra information of tissue, i.e., analyte (i.e., glucose). It may be associated to a concentration of analyte, i.e., blood sugar. Long wavelength infrared energy (i.e., thermal) emitted by a person is monitored, and is used as an infrared energy source to carry out infrared absorption measurement of a specific component of the blood in infrared absorption wavelength characteristics on the components. When a signal ratio is taken, in the state that the measurement is synchronized with a cardiac cycle of heart systole and diastole, signal distribution caused by a (not beating) vein or tissue may be cancelled. A temperature sensing unit for measuring an internal temperature of an arm or other vascular organs of a person is utilized to adjust the measurement of component concentration against a temperature depending influence.

The alpha-wave inducer will now be described.

### Embodiment 1

First, the user puts on the electric stimulating unit 20, and pushes down the electric power key 106 to initialize the system. The user sets frequency, voltage, cycle and operation time, respectively, by use of the mode selecting key SET. When selecting the wire/wireless mode, the microprocessor 120 automatically sets the wire/wireless mode according to whether the jack 202 is inserted in the connector 128. Specifically, if the jack 202 is inserted, it proceeds to the wire mode, while if the jack 202 is not inserted, it proceeds to the wireless mode. The mode selection is implemented by the microprocessor 120 according to the voltage value detected by the non-contact detector 126. The control of the applied voltage according to the wet state of the user is similar to the above process, the detailed description of which will be omitted here. Since the control of the applied voltage according to the wet state of the user is implemented in the state where the jack 202 is inserted into the connector 128, the control is preferably implemented under the wire mode. The frequency and voltage set by the user are applied to the user's ears every set cycle, and the inducer operates during the set operation time. If the user releases the connection between the jack and the connector 128, the microprocessor 120 determines the release from the connector 128, which is connected to one terminal of the microprocessor 120, to implement the switching of the wireless mode from the wire mode. At the same time, the microprocessor 120 transmits the set value set by the user to the transmitter/receiver 216 of the electric stimulating unit 20 through the transmitter/receiver 130. When the jack 202 is detached from the connector 128, the wire/wireless detector 212 of the electric stimulating unit 20 detects the release of the jack 202 and initializes the system. At that time, considering the time required for the initializing process of the electric stimulating unit 20, the setting data is transmitted from the transmitter/receiver 130. The data received by the transmitter/receiver 216 is read by the remote control unit 218, and the electric power supply 220 outputs the corresponding voltage. The oscillator 222 outputs the corresponding frequency to apply it to a contact terminal 214. In other words, the electric stimulation is applied to the auricle of vagus nerves to induce 7Hz to 14Hz of alpha-waves.

If external stimulation is applied to brain, reticula formation acts, and a conscious focus is externalized, and an alpha-wave interruption effect occurs. On the other hand, a thalamus system diffuses alpha-waves in a wide range in such a way that the conscious focus is internalized, attention power and concentration of concern are controlled, and the function of corpus callosum is activated only in the state of low brain waves such as alpha-waves, many information exchange between left and right brain occurs and an ability for receiving new information such as a learning ability is improved. Thus, through alpha-waves applied to the auricle of an ear from the electric stimulating unit 20 according to the present invention, keeping of power of concentration is maximized for a long time, a long-term memory is enhanced, and a thinking procedure in which the previous information is combined is smoothly performed.

In addition, an alpha-wave of brain waves is a brain wave that appears in the state where an awakening level is not too high and not too low. Due to the alpha-wave, the state of brain is comfortably induced and the comfortable state of brain is maintained. If the alpha-waves generated in the electric stimulating unit 20 are applied to the auricle of an ear, due to induction of alpha-waves, an outside-oriented awakening state is prevented from being excessive and induction of alpha-waves helps to smoothly make selective attention concentration of brain.

### Embodiment 2

A manual mode directly set by the user is described in the first embodiment, while an automatic mode set by the user will now be described in the second embodiment.

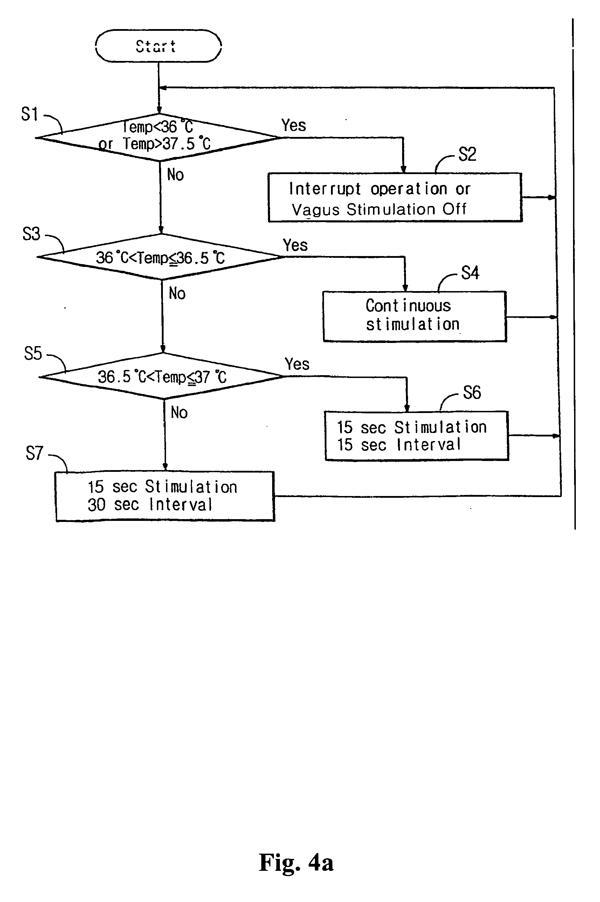

If the automatic mode is selected by the user, the inducer operates according to the initially set frequency, voltage, cycle and operation time. As in the first embodiment, the microprocessor 120 automatically sets the wire/wireless mode according to whether the jack 202 is inserted in the connector 128. It is preferable to use the wire mode of which the jack 202 is inserted, so as to provide the wet state of the user. Even though a wet state of the user cannot be checked, data regarding body temperature and blood sugar level is transferred to the microprocessor 120 through the transmitter/receiver, in the case of the wireless mode. In any case, the inducer operates according to the value set as an initial value, and the body temperature/blood sugar detector alternatively checks the body temperature and the blood sugar in order. The body temperature and the blood sugar are input to the microprocessor 120 through the jack 202. The microprocessor 120 converts the electric stimulation to the optimum applying state by use of a program itself (which can be set by a manufacturer). At that time, parameter converting it to the optimum state is the body temperature or the blood sugar or a combination thereof, and it is properly shown in Figs. 4a to 4c.

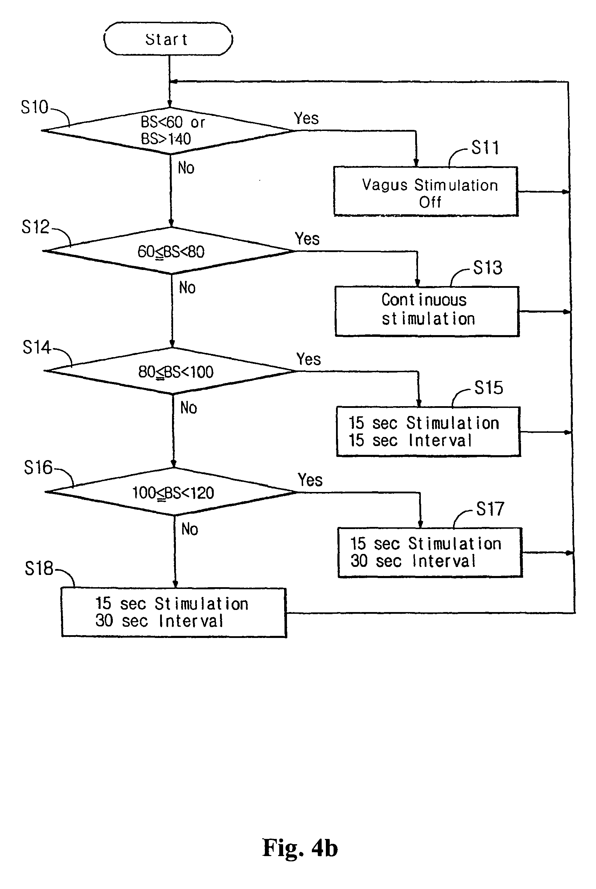

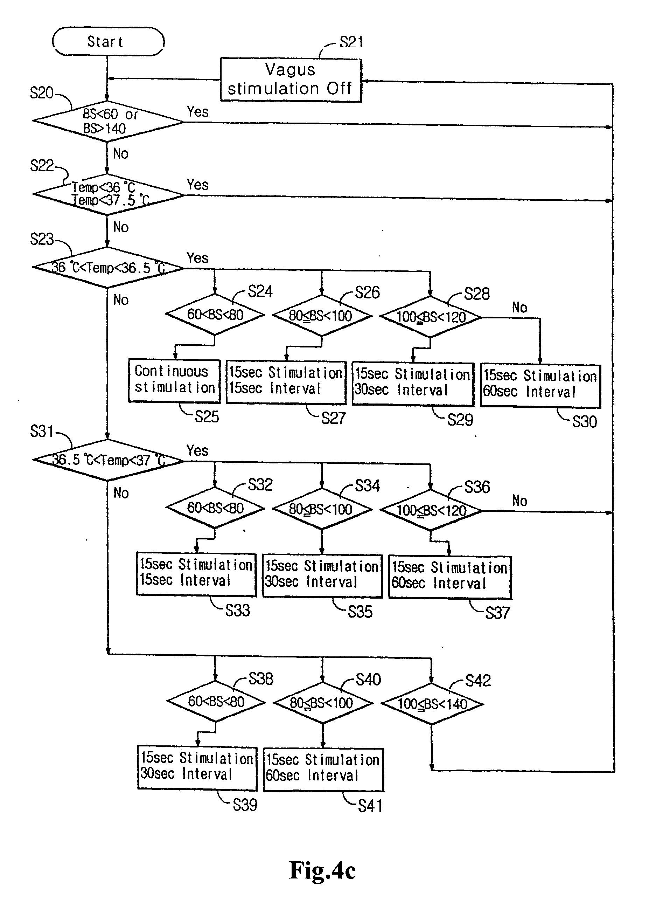

Fig. 4a is a flowchart depicting a stimulating method according to the body temperature, Fig. 4b is a flowchart depicting a stimulating method according to the blood sugar, and Fig. 4c is a flowchart depicting a stimulating method according to the body temperature and the blood sugar.

As shown in Fig. 4a, as the result that the data transmitted from the body temperature/blood sugar detector 209 is analyzed, if the body temperature 'Temp' is less than 36°C or more than 37.5°C (step S1), the microprocessor 120 interrupts the application of the voltage to the user (step S2). Then, if the body temperature is less than 36°C or more than 37.5°C by again pushing down the automatic mode button or implementing the check of the body temperature by preset times, the electric power is automatically turn off to turn off the system. If the body temperature is not less than 36°C or not more than 37.5°C, the body temperature is subdivided (steps S3 and S5) to apply the corresponding electric stimulation (steps S4, S6 and S7). The application of stimulation is continuously implemented in the unit of second or is periodically implemented.

As shown in Fig. 4b, as the microprocessor 120 analyzes the data transmitted from the body temperature/blood sugar detector 209, if blood sugar level 'BS' is less than 60 mg/dL or more than 140 mg/dL (step S 10), the voltage application to the user is interrupted (step S11). Then, if the blood sugar level 'BS' is less than 60 mg/dL or more than 140 mg/dL by again pushing down the automatic mode button or implementing the check of the blood sugar by preset times, the electric power is automatically turn off to turn off the system. If the blood sugar 'BS' is not less than 60 mg/dL or not more than 140 mg/dL, the blood sugar is subdivided (steps S12, S14 and S16) to apply the corresponding electric stimulation (steps S13, S15, S17 and S18). The application of stimulation is continuously implemented in the unit of second or is periodically implemented.

As shown in Fig. 4c, when analyzing the data transmitted from the body temperature/blood sugar detector 209, the body temperature/blood sugar detector detects the body temperature 'Temp' and the blood sugar 'BS' according to the program control of the microprocessor 120 by turns. As shown in the figures, if the blood sugar is less than 60 mg/dL or more than 140 mg/dL (step S20), the voltage application to the user is interrupted (step S21). Then, if the blood sugar is not less than 60 mg/dL or not more than 140 mg/dL, the body temperature is detected. If the body temperature is less than 36°C or more than 37.5°C (step S22), the microprocessor 120 interrupts the application of the voltage to the user (step S21). Then, if the body temperature is not less than 36°C or not more than 37.5°C, the microprocessor determines that the body temperature and the blood sugar are a normal state. The control of the voltage application is implemented every body temperature (steps S23 and S31) and every blood sugar (steps S24, S26, S28, S32, S34, S36, S38, S40 and S42). Accordingly, the electric stimulation is applied to the auricle of vagus nerves to induce 7Hz to 14Hz of alpha-waves.

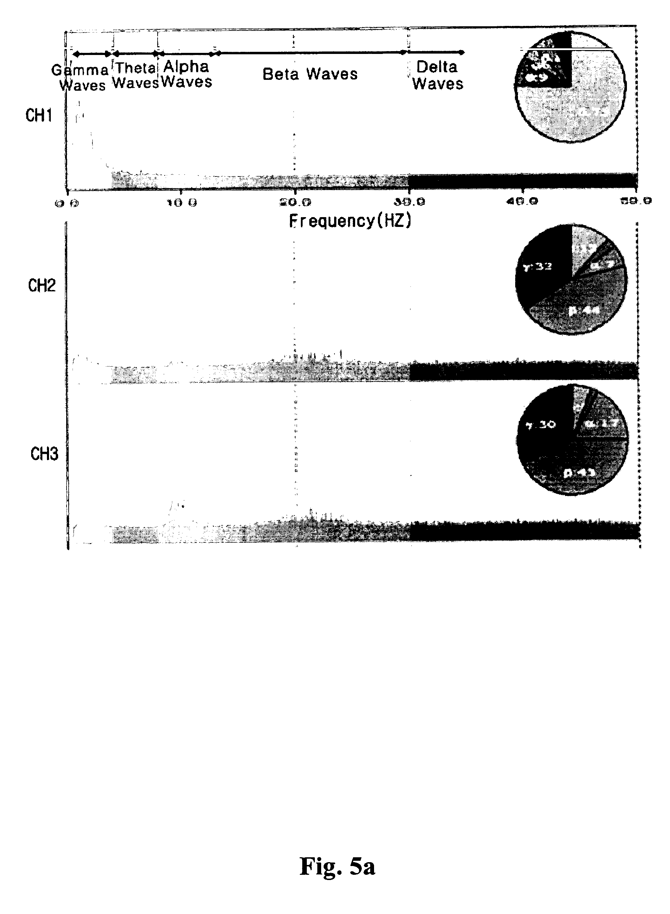

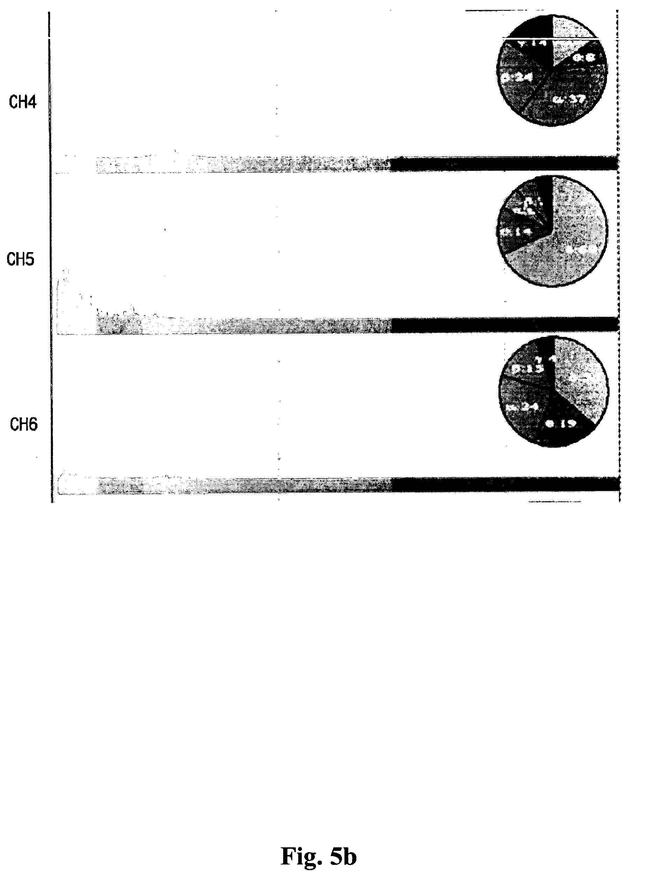

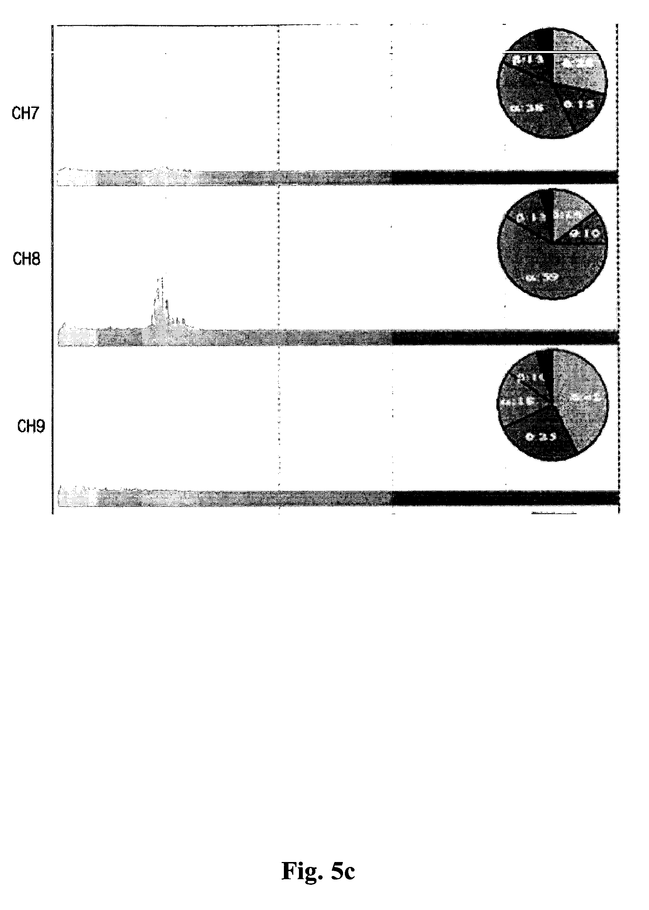

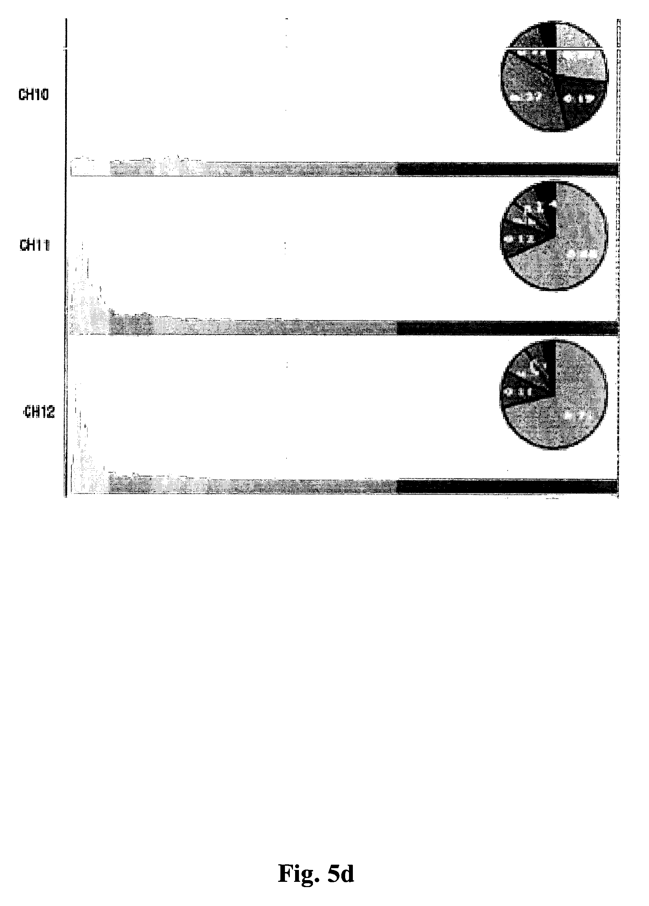

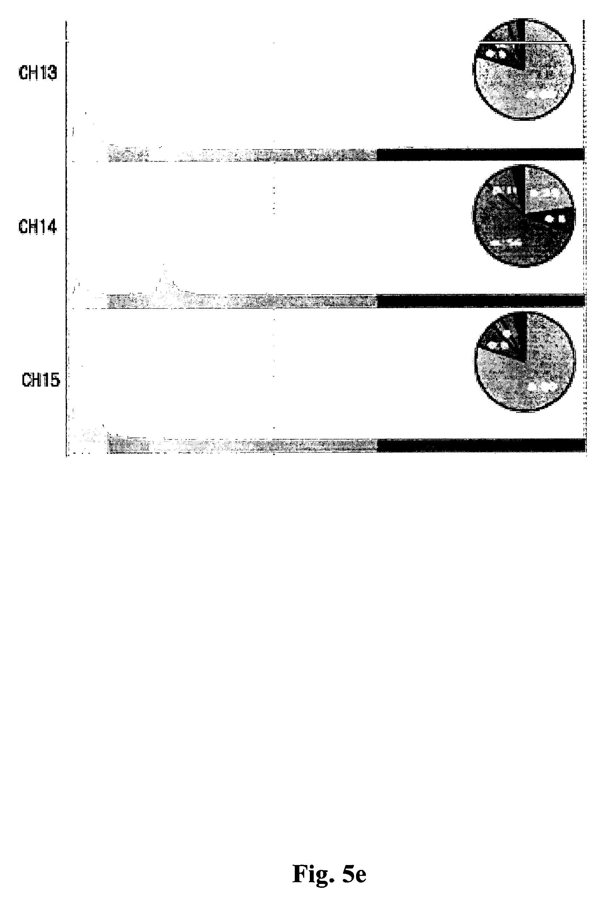

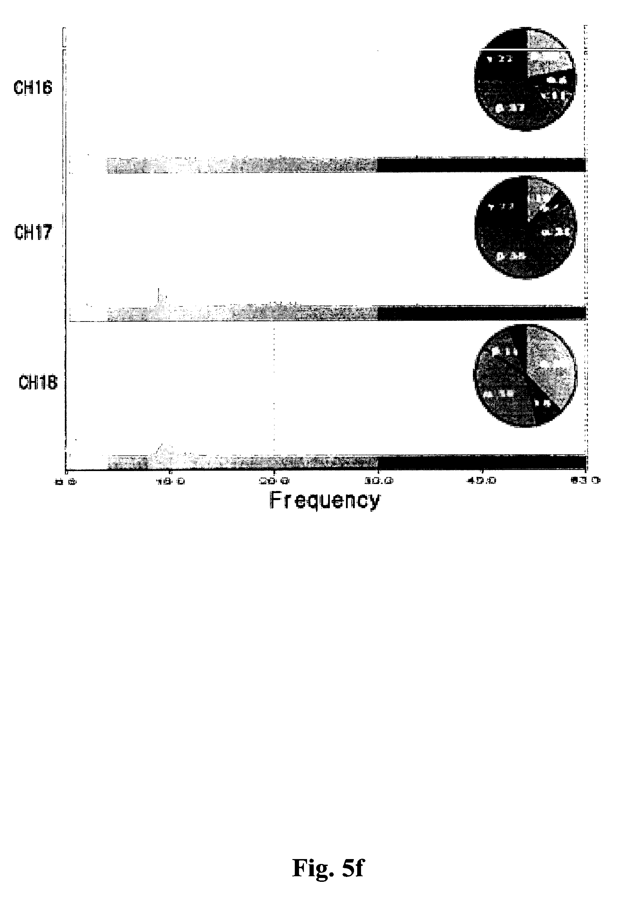

Figs. 5a to 5f are graphs showing distribution the of alpha waves, beta waves, gamma waves, delta waves, and theta waves before using an alpha-wave inducer according to the present invention. Referring to Figs. 5a to 5f, the alpha (α) waves, beta (β) waves, gamma (γ) waves, delta (δ) waves, and theta (θ) waves and its distribution are quantitatively depicted, respectively, which are results measured at eighteen regions (ch1 to ch18) of the head. It is understood from the figures that different distributions are formed from different regions of the head.

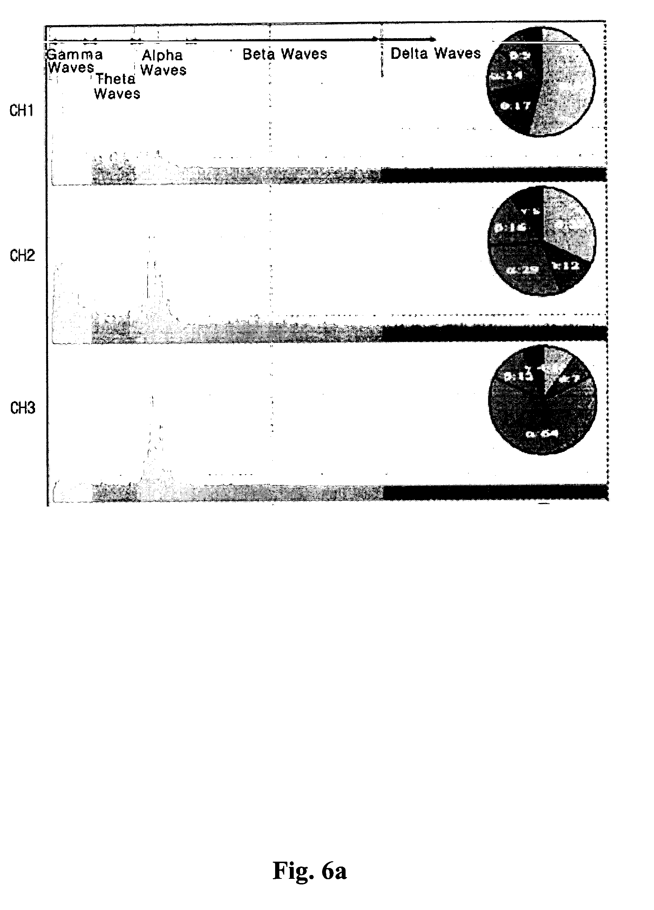

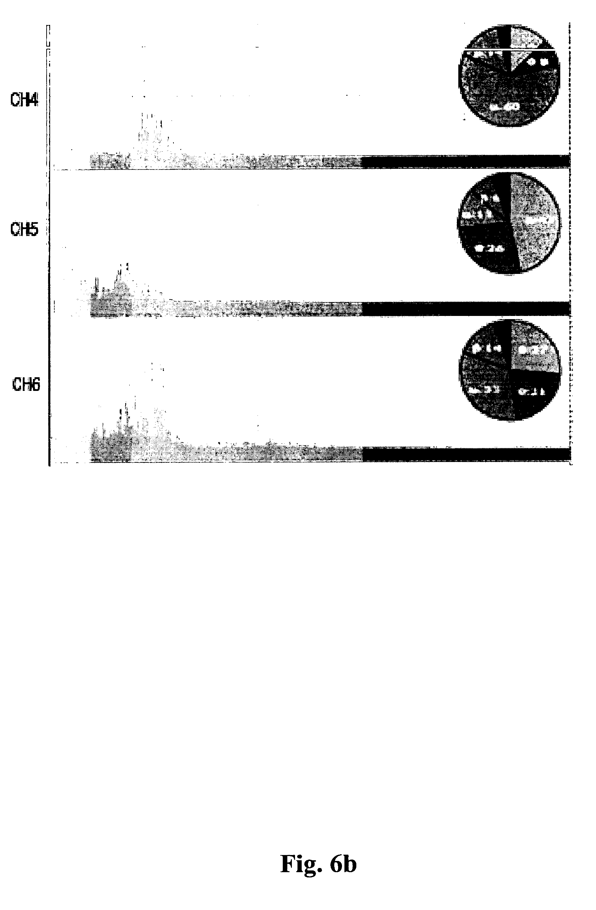

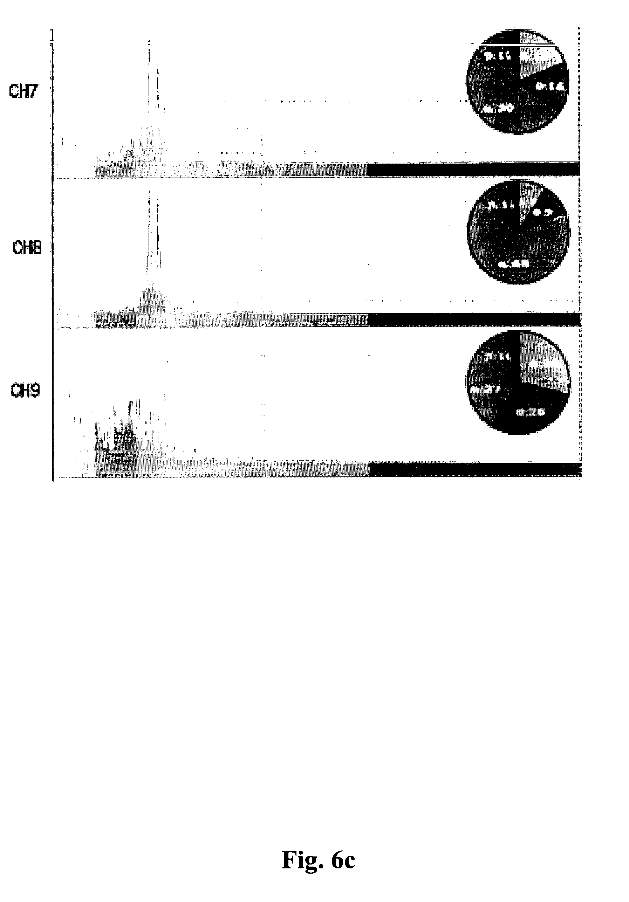

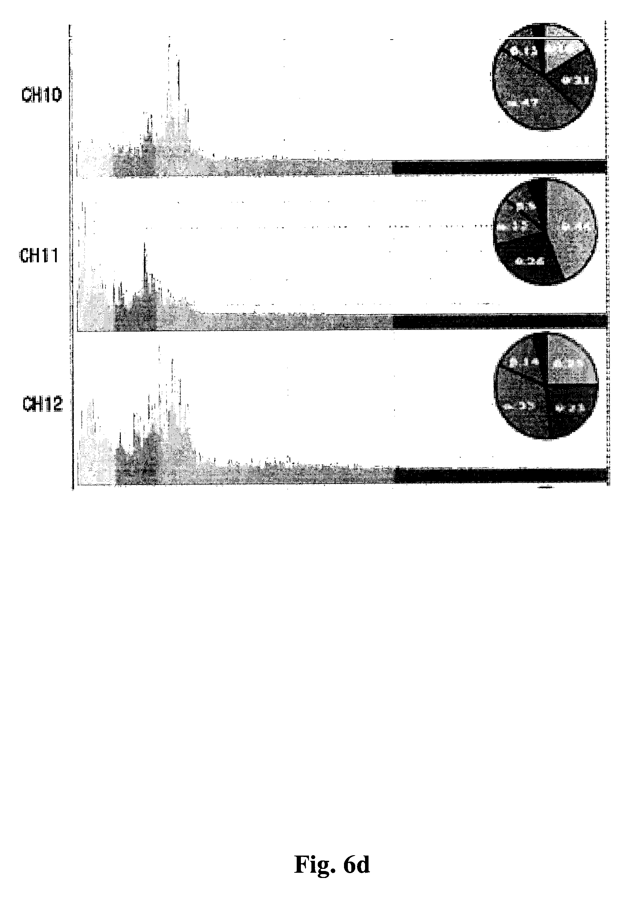

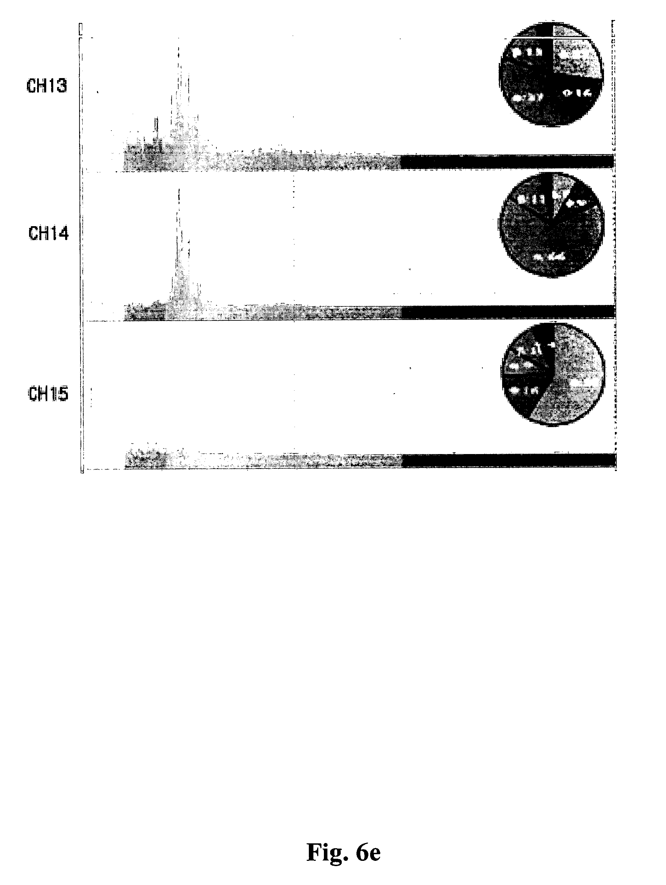

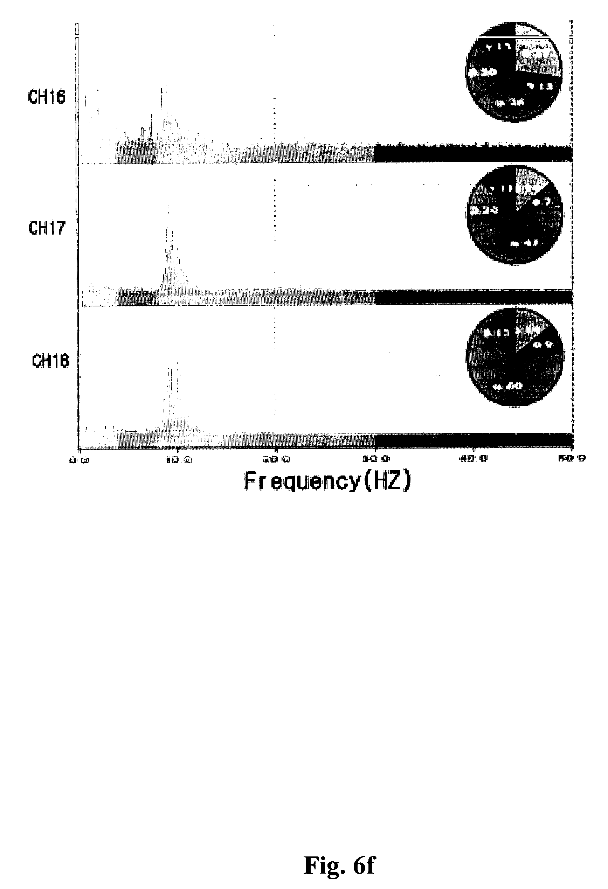

Figs. 6a to 6f are graphs showing distribution of alpha waves, beta waves, gamma waves, delta waves, and theta waves after use of the alpha-wave inducer according to the present invention. It would be understood from the figures that quantitative distribution of the alpha-waves is increased in eighteen regions of the head, respectively, after applying the electric stimulation by use of the inducer.

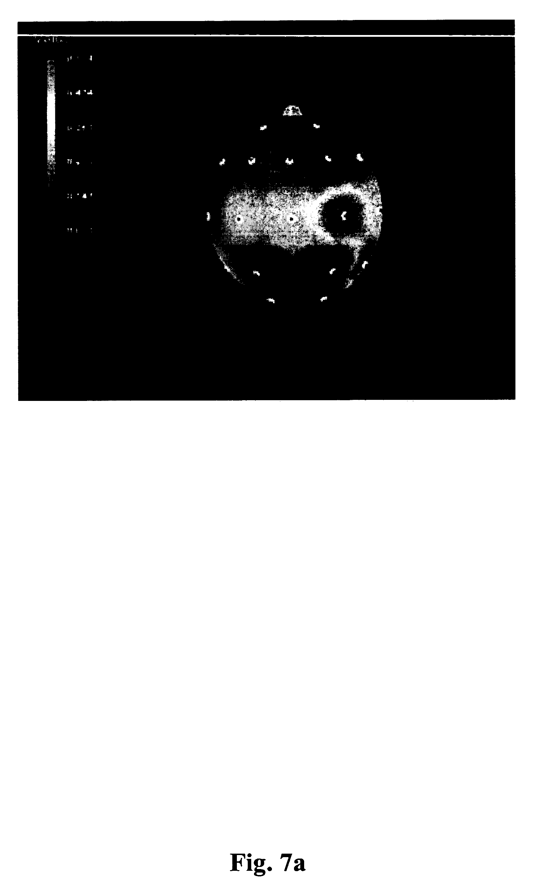

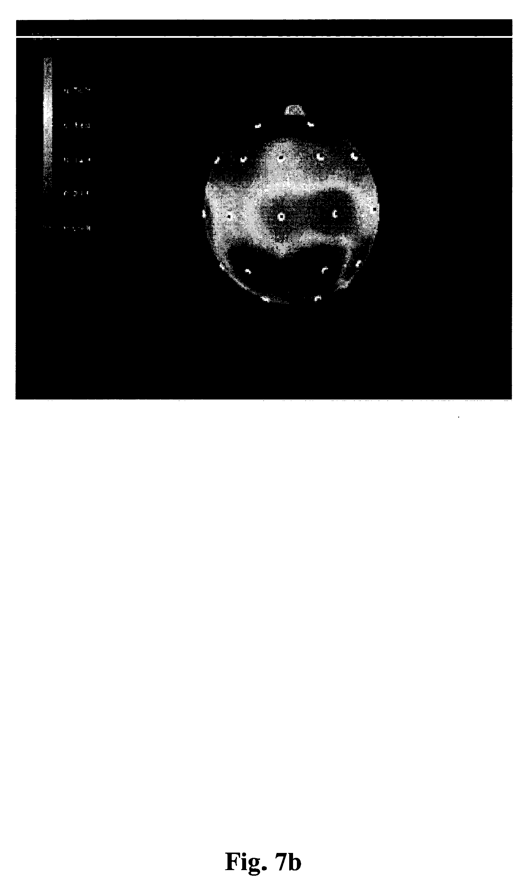

Figs. 7a and 7b are views illustrating the state which alpha power is increased in a head before and after use of the alpha-wave inducer according to the present invention. It would be understood from the figures that eighteen regions of the head are designated and the alpha-waves is increased after applying stimulation.

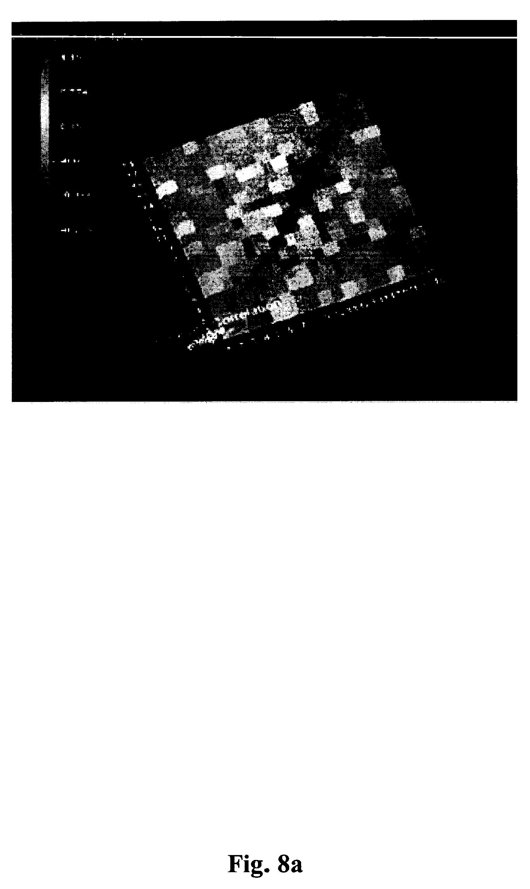

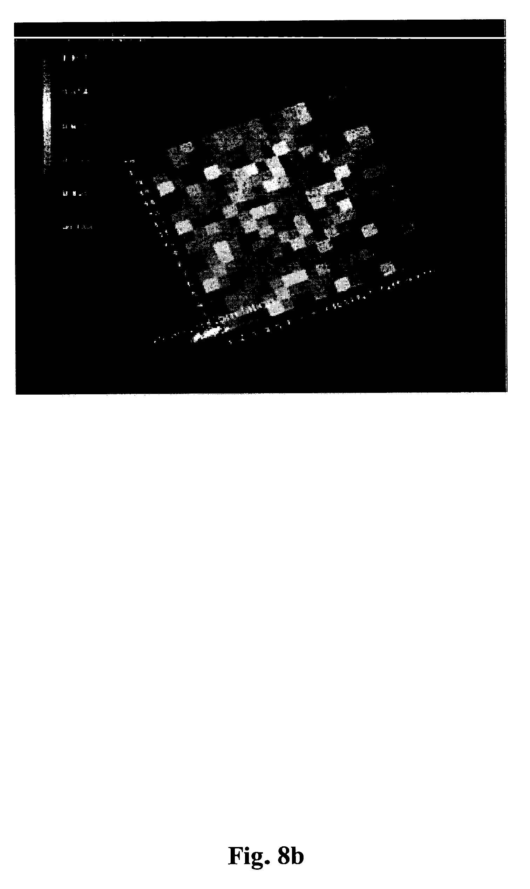

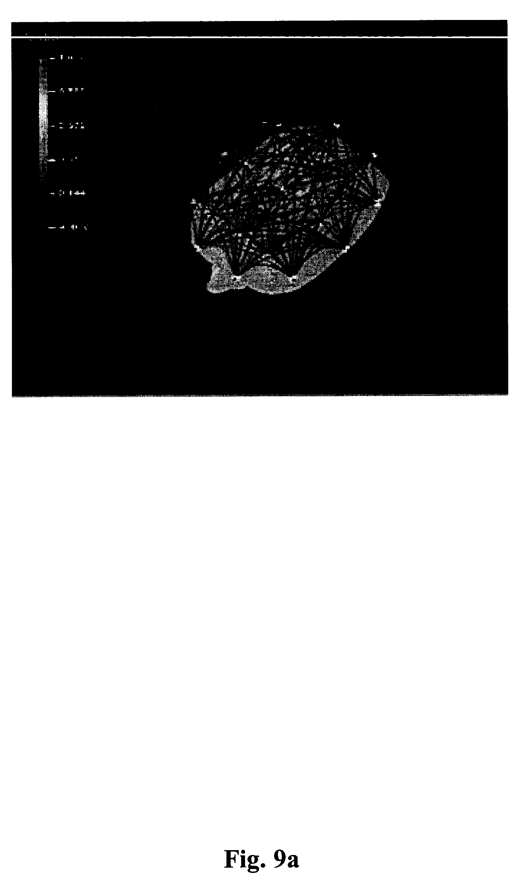

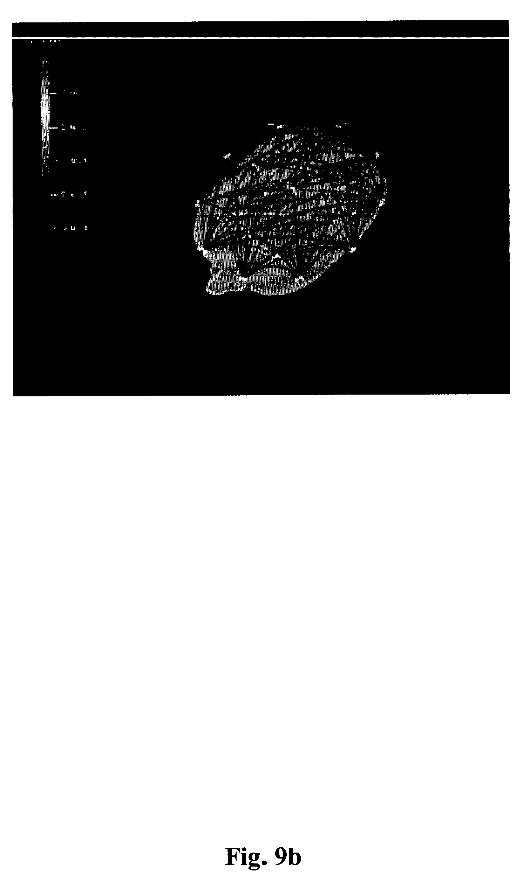

Figs. 8a and 8b and Figs. 9a and 9b are views illustrating the state which synchronization is increased in a forebrain before and after use of the alpha-wave inducer according to the present invention. It would be understood from the figures that the synchronization of the brain cell related to the respective regions of the brain is increased after applying stimulation by use of the electric stimulation.

The term "synchronization" herein refers to the case where a plurality of nerve cells activate synchronously. For example, a waveform of a low frequency having high amplitude when the eyes close is changed to a waveform of a high frequency having low amplitude when the eyes open, and is again changed to a waveform of a low frequency when the eyes close. Rhythm of the low frequency can be easily seen in the state that a health adult is emotionally stable, with his/her eyes closing. It can be clearly seen in occipital lobes, and the frequency component is 7 Hz to 14 Hz (it's average is 10 Hz). Almost the same wave (amplitude, frequency, phase) is represented in most of the records, and it is called synchronized EEG.

Accordingly, the increase of the synchronization of the brain cells to the respective regions after applying stimulation is a measure indicating the fact that the activation of the alpha-waves is increased to all regions of the brain.

An alpha-wave inducer according to another embodiment of the present invention will now be described in detail with reference to the accompanying drawings.

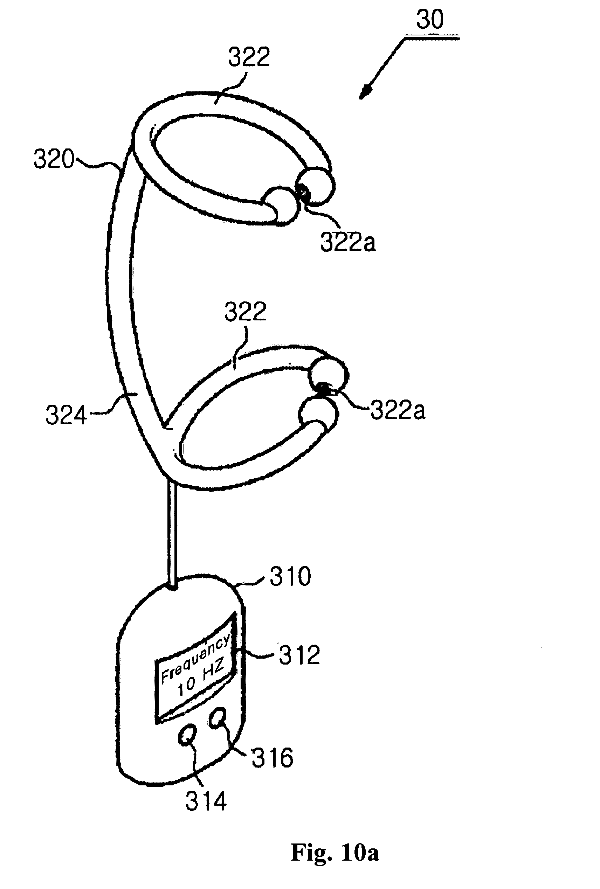

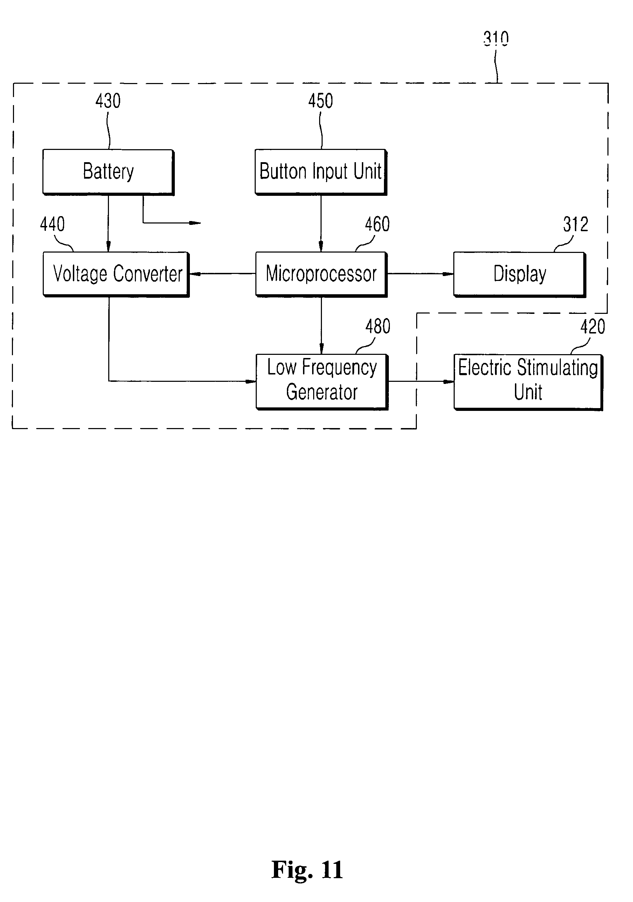

As illustrated in Figs. 10a to 10c and Fig. 11, another preferred embodiment of the alpha-wave inducer according to the present invention is implemented from the embodiment of the present invention illustrated in Figs. 1 to 3 but consists of only the construction for providing a low frequency and low voltage to an ear without checking the user's humidity, body temperature and blood sugar.

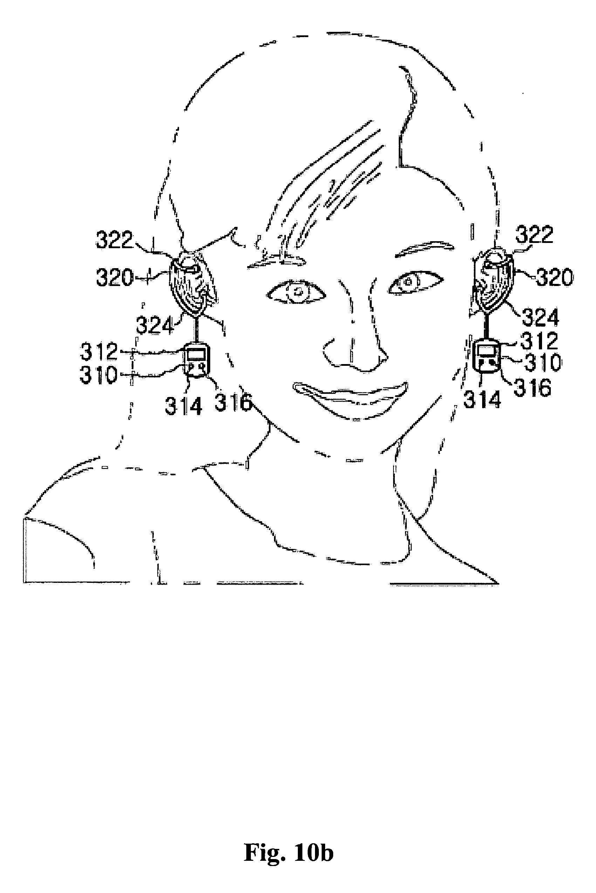

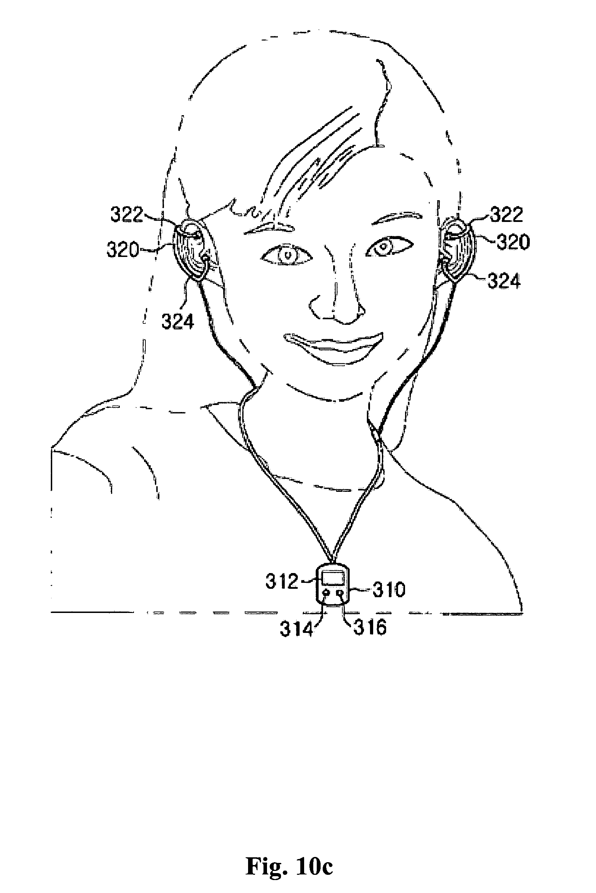

Referring to Figs. 10a and 11, an alpha-wave inducer 30 according to another embodiment of the present invention mainly includes a main control unit 310 and an electric stimulating unit 320 that acts on auricle of an ear. The main control unit 310 is implemented as a pendant having an ear ring shape illustrated in Fig. 10b or a pendant having a necklace shape illustrated in Fig. 10c and includes a battery 430, a voltage converter 440, a low frequency generator 480, a button input unit 450, a display 312, and a microprocessor 460.

The button input unit 450 includes an electric power/function button 314 and a setting button 316 and the display 312 for displaying a value set by an operation of the button input unit 450 are disposed outside a housing of a pendant in which the main control unit 310 is implemented. Preferably, the display 312 is implemented as a liquid crystal display (LCD). Here, the shape of the pendant housing may be changed according to a user's taste or a manufacturer's design technology and the scope of the present invention is not limited to a pendant having a specific shape.

Elements such as the battery 430, the voltage converter 440, the low frequency generator 480, and the microprocessor 460 are disposed inside the housing of the pendant.

The battery 430 supplies an electric power required for operations of other elements, and the voltage converter 440 is controlled by the microprocessor 460 and converts the electric power from the battery 430 into a voltage having a predetermined value set at 0 mV to 100 mV

The low frequency generator 480 is controlled by the microprocessor 460 and generates a low frequency of a predetermined value set at 1 Hz to 50 Hz based on the voltage from the voltage converter 440. That is, the low frequency generator 480 includes an oscillator (not shown) and converts a high frequency pulse from the oscillator into a frequency set in the microprocessor 460, thereby generating an electric pulse of a frequency wanted by the user.

The microprocessor 460 controls the frequency of the low frequency generator 480 and the voltage of the voltage converter 440 based on a value set by an operation of the button input unit 450. That is, the microprocessor 460 includes a plurality of setting modes such as a frequency setting mode, a voltage setting mode, a cycle setting mode, and an operation time setting mode. Thus, the microprocessor 460 turns on an electric power if the electric power/function button 314 is clicked once in the state where the electric power is turned off, and if the electric power/function button 314 is continuously clicked in the state where the electric power is turned on, the microprocessor 460 converts a setting mode into other functions in the sequence of the frequency setting mode, the voltage setting mode, the cycle setting mode, and the operation time setting mode, and if the electric power/function button 314 is double- clicked, the microprocessor 460 turns off an electric power. Here, the type and sequence of setting modes are illustrative and thus may be easily changed bv one of ordinary skill in the art. When the user presses the setting button 316 in the state where a specific setting mode is defined, the setting mode is converted from the currently-set value in an ascending or descending order in the unit of predetermined time (for example, 0.2 seconds). In case where it is determined that setting values are converted in an ascending order, if a set value reaches a maximum value in a set range, a minimum value is again converted in an ascending order.

Meanwhile, the electric stimulating unit 320 includes a plurality of contact terminals 322a connected to the main control unit 310 via electric wires and applies a low frequency generated by the low frequency generator 480 and a low voltage generated by the voltage converter 440 to the auricle of an ear. Preferably, the electric stimulating unit 320 has at least one ear ring shape in which an ear is pressed at both sides. In case where the electric stimulating unit 320 includes a plurality of ear rings 322, the respective ear rings 322 are combined in a support 324 and are formed as one body. A portion of each ear ring 322 that presses skin at both sides of an ear is rounded, and the contact terminals 322a formed of metallic pieces are installed in the middle of the ear ring 322. Here, the shape of the electric stimulating unit 320 is just one embodiment and thus may be easily changed by one of ordinary skill in the art.

The alpha-wave inducer 30 according to embodiments of the present invention will now be described.

First, when the user turns on an electric power by once clicking the electric power/function button 314 installed outside the main control unit 310 implemented as an ear ring or necklace-shaped pendant, the microprocessor 460 is first set in a frequency setting mode. In the frequency setting mode, the microprocessor 460 displays a currently-set frequency setting value on the display 312. The user can change a frequency setting value by pressing the setting button 316. At this time, preferably, a frequency value is set to one value selected from a range of 7 Hz to 14 Hz. The changed setting value may be checked on the display 312. When the user clicks the electric power/function button 314 again, the setting mode of the microprocessor 460 is converted into a voltage setting mode. In the same way, the setting mode of the microprocessor 460 may be converted into a cycle setting mode or an operation time setting mode. In each setting mode, a corresponding setting value may be changed using the setting button 316.

If setting values are defined, the microprocessor 460 controls the frequency of the low frequency generator 480 and the voltage of the voltage converter 440 according to setting values and generates an electric pulse wanted by the user. The generated electric pulse is applied to the plurality of contact terminals 322a included in the electric stimulating unit 320 via electric wires. Thus, when the user puts on the electric stimulating unit 320 having the shape of the ear ring 322, the auricle of vagus nerves of an ear are stimulated by the electric pulse generated by the main control unit 310.

The invention is not restricted to the features of the described embodiments.

With the above description, the alpha-wave inducer according to embodiments of the present invention may treat hypertension, circulatory illnesses, diabetes, adiposity, hyperlipemia, mania and hypochondria, anxiety disorders, memory deficits (i.e., dementia and Alzheimer's diseases), attention deficits, neuropathy encephalopathy, epilepsy, tremor and metal disorders, chronic arthritis and pains, sleep apnea and respiratory disorders, withdrawal symptoms from maternal use of drugs, disorders associated substance abuse, digestive system disorders, growth disorders, menopausal disorders, autoimmune disorders, malignant cancel of organs dominated by vagus nerves, and disorders resulted from hebetation of parasympathetic system (i.e., senescence), according to the applied frequency and voltage. Furthermore, power of attention and power of concentration can be improved by relieving mental tension and fatigue, and stress can be reduced to keep the optimum brain state, and a memory can be enhanced, and a thinking procedure in which the previous information is combined can be improved.

According to embodiments of the present invention, the alpha-wave inducer can be implemented as an accessory such as an ear ring or a necklace such that the auricle of vagus nerves of an ear is directly stimulated for a long time.

## Claims

1. An alpha-wave inducer comprising:
a main control unit for generating a low frequency signal of frequency and cycle selected from a range of 1 Hz to 50 Hz and a voltage selected from a range of 0 mV to 100 mV to induce an alpha-wave; and
an electric stimulating unit arranged to apply the low frequency signal generated in the main control unit to an auricle of vagus nerves of an ear.

2. The alpha-wave inducer as claimed in claim 1, wherein the main control unit is arranged to generate a low frequency signal of frequency and cycle selected from a range of 7 Hz to 14 Hz and a voltage selected from a range of 1 mV to 20 mV.

3. The alpha-wave inducer as claimed in claim 2, wherein the electric stimulating unit is arranged to press an ear at both sides, and the main control unit is arranged to suspend on the electric stimulating unit via a connecting line and has an ear ring shape.

4. The alpha-wave inducer as claimed in claim 2, wherein the electric stimulating unit is arranged to presses an ear at both sides, and the main control unit is arranged to connect to the electric stimulating unit via a connecting line, and the connecting line has a necklace shape.

5. The alpha-wave inducer as claimed in one of claims 1 to 4, wherein the main control unit comprises:
a key input unit for supporting user operation by system initialization when an electric power is supplied;
a microprocessor for setting a frequency, a voltage, a cycle and an operation time according to a key input signal transferred from the key input unit, for controlling an output of the frequency and voltage according to the set, for determining whether the outputted frequency and voltage are applied to an ear of a user, for performing a switching of a wire/wireless mode by determining whether a jack is connected to an electric stimulating unit, for outputting a transmitting signal to wirelessly transmit a setting data after a predetermined time, when the wireless mode is switched, and for outputting a turn-on/off signal corresponding to an alarm signal and the turn-on/off signal indicative of a wire/wireless mode control state; a control-side transmitter/receiver for transmitting the setting data according to the transmitting signal and transmitting/receiving input/output data; an oscillator for oscillating at a frequency of 1 Hz to 50 Hz according to a frequency output control; and a connector connected to the jack and connected to the microprocessor for determining the wire/wireless state according to variations of input current value;
a connecting line arranged to connect to the connector with the jack and transfer the frequency and voltage from the jack to the electric stimulating unit; and
the electric stimulating unit including: a contact terminal arranged to connect to the connecting line and contacted to an auricle of vagus nerves of the ear; a wire/wireless detecting unit interposed between the connecting line and the contact terminal arranged for detecting the wire/wireless mode state according to whether it is connected to the jack or not; a remote control unit arranged to determine the wire/wireless mode state, for controlling operation of the electric stimulating unit itself, in case of the wireless mode, and for controlling the frequency and voltage according to the set data transmitted from the control-side transmitter/receiver; a transmitter/receiver arranged to communicate with the control-side transmitter/receiver for transmitting the input/output data to the remote control unit; an oscillator arranged to supply the frequency to the remote control unit; and a battery arranged to supply an electric power to the remote control unit.

6. The alpha-wave inducer as claimed in claim 5, wherein the main control unit further comprises a non-contact detector arranged to detect whether the electric stimulating unit is non-contacted to the ear by use of charge accumulation flowing through the jack of the control unit to output the result to the microprocessor.

7. The alpha-wave inducer as claimed in claim 6, further comprising, in order to prevent electric short circuit when the contact state based on the signal transmitted from the non-contact detecting unit is determined,
a variable resistor R1 arranged to form a closed circuit together with the contact terminal for performing a voltage drop according to a resultant resistance value;
a voltage comparator COM arranged to compare a voltage generated by the resultant resistance value with a predetermined reference voltage;
a switch TR1 arranged to switch according to the comparison results of the voltage comparator COM;
a capacitor C2 arranged to discharge by the switch TR1 to transmit a signal notifying whether normality or not to the microprocessor; and
an electric power control unit, which it determines as the normality when the capacitor C2 is discharged, and interrupts supply of a direct current, and which only the voltage drop happens by the variable resistance R1 when the capacitor C2 is not discharged.

8. The alpha-wave inducer as claimed in claim 5, wherein the main control unit further comprises a display arranged to display a set value according to a key input signal transmitted from the key input unit, and for outputting or lighting an operation state.

9. The alpha-wave inducer as claimed in claim 5, wherein the electric stimulating unit has a body temperature and blood sugar detecting unit made in a shape like a crater of a gently slant ridgeline to be easily inserted in an external auditory meatus, and arranged so as to detect a signal for determining body temperature and blood sugar using an infrared processing module, and the body temperature detecting unit has a drilled top surface H to receive an infrared signal.

10. An alpha-wave inducer comprising:
a battery arranged to supply electric power to the entire of a system;
a voltage converter arranged to convert the electric power from the battery into a voltage of a predetermined value set at 0 mV to 100 mV;
a low frequency generator arranged to generate a low frequency of a predetermined value set at 1 Hz to 50 Hz based on the voltage from the voltage converter;
a button input unit including an electric power/function button and a set button;
a display arranged to display a value set by an operation of the button input unit;
a microprocessor arranged to control a frequency of the low frequency generator and a voltage of the voltage converter based on the value set by the operation of the button input unit; and
an electric stimulating unit including a plurality of contact terminals arranged to apply the low frequency generated by the low frequency generator to an auricle of an ear.

11. The alpha-wave inducer as claimed in claim 10, wherein the microprocessor is arranged to turn on an electric power if an electric power/function button is clicked once in the state where the electric power is turned off, and if the electric power/function button is continuously clicked in the state where the electric power is turned on, the microprocessor converts a setting mode into other functions, and if the electric power/function button is double- clicked, the microprocessor turns off an electric power, and if a setting button is pressed, the microprocessor converts a currently-set value in an ascending or descending order in a unit of predetermined time.

12. The alpha-wave inducer as claimed in claim 10, wherein the electric stimulating unit has an ear ring shape in which an ear is pressed at both sides.

13. The alpha-wave inducer as claimed in claim 10, wherein the electric stimulating unit has a necklace shape.

14. The alpha-wave inducer as claimed in claim 10, wherein the electric stimulating unit comprises a plurality of ear rings for pressing an ear at both sides and a support for supporting the plurality of ear rings.
